Europäisches Patentamt

European Patent Office

Office européen des brevets

(11) Numéro de publication: **0 350 392**
A2

# (12) DEMANDE DE BREVET EUROPEEN

(21) Numéro de dépôt: **89401916.5**

(22) Date de dépôt: **04.07.89**

(51) Int. Cl.⁵: **C 12 Q 1/68**
C 12 Q 1/10, C 07 H 21/02,
C 07 H 21/04

(30) Priorité: **05.07.88 FR 8809076**

(43) Date de publication de la demande:
**10.01.90 Bulletin 90/02**

(84) Etats contractants désignés:
**AT BE CH DE ES FR GB GR IT LI LU NL SE**

(71) Demandeur: **IRE-MEDGENIX S.A.**
**B-6220 Fleurus (BE)**

(72) Inventeur: **Moureau, Philippe**
**12, Rue des Cévennes**
**B-5890 Chaumont-Gistoux (BE)**

**Derclaye, Isabelle**
**Chaussée de la Hulpe, 184 (Bte 6)**
**B-1170 Bruxelles (BE)**

**Delor, Isabelle**
**Avenue Marcel Tilquin 5**
**B-1330 Rixensart (BE)**

**Cornelis, Guy**
**Avenue des Combattants 2bis**
**B-1950 Kraainem (BE)**

(74) Mandataire: **Warcoin, Jacques et al**
**Cabinet Régimbeau 26, avenue Kléber**
**F-75116 Paris (FR)**

(54) Sondes d'acides nucléiques utiles pour détecter de manière spécifique différentes espèces bactériennes du genre campylobacter.

(57) La présente invention a pour objet des sondes d'acides nucléiques ADN ou ARN utiles pour détecter de manière spécifique l'une des espèces de bactéries du genre Campylobacter consistant en l'espèce Campylobacter coli, l'espèce Campylobacter jejuni, l'espèce Campylobacter fetus, l'espèce Campylobacter laridis et l'espèce Campylobacter Upsaliensis, directement par hybridation avec l'ADN ou l'ARN de cette espèce ou par une méthode RFLP

EP 0 350 392 A2

Bundesdruckerei Berlin

**Description**

## SONDES D'ACIDES NUCLEIQUES UTILES POUR DETECTER DE MANIERE SPECIFIQUE DIFFERENTES ESPECES BACTERIENNES DU GENRE CAMPYLOBACTER

Le domaine technique de la présente invention est celui des sondes constituées par des séquences d'acides nucléiques, ADN mono-brin ou ARN, marquées.

L'hybridation ADN-ADN est une technique essentielle en biologie moléculaire. Son potentiel d'application industrielle, notamment en santé humaine dans le domaine du diagnostic des maladies infectieuses, est largement reconnu. Dans ce domaine d'application particulier, la méthode repose sur l'utilisation de sondes à ADN, c'est-à-dire de fragments d'ADN marqués spécifiques de micro-organismes donnés, comme outil spécifique d'identification.

Pour de nombreux micro-organismes, les méthodes d'identification communément utilisées à ce jour en routine médicale restent basées sur la culture en milieu "spécifique" associée à certains tests biochimiques. Ces méthodes ne permettent souvent l'obtention d'une réponse qu'après plusieurs jours, et restent parfois entachées d'un manque de spécificité. Ceci est particulièrement vrai pour les espèces de Campylobacter (jejuni, coli), qui représentent la première cause des gastro-entérites bactériennes aiguës en pays développés.

Le choix de la technique ADN-ADN pour la détection de micro-organismes, pourrait donc s'appliquer en priorité à l'identification, dans les selles ou dans la nourriture, d'agents pathogènes telles que les souches de Campylobacter responsables de gastro-entérite.

La présente invention concerne des sondes d'acides nucléiques pour la détection et l'identification de micro-organismes bactériens du genre Campylobacter (ci-après abrégé par C.)

Plus précisément la présente invention a pour objet des sondes d'acides nucléiques ADN ou ARN consistant en des séquences déterminées d'ADN ou ARN respectivement marquées, utiles pour détecter et identifier de manière spécifique les différentes espèces bactériennes du genre Campylobacter.

En particulier, la présente invention a pour objet des sondes spécifiques de l'espèce Campylobacter coli et d'autres sondes spécifiques de l'espèce Campylobacter jejuni.

En effet, parmi les espèces de Campylobacter responsables d'entérites, Campylobacter jejuni et Campylobacter coli représentent ensemble 99 % des cas, alors que C. laridis et C. fetus ne représentent que 1 % des cas. Le pourcentage des C.jejuni par rapport aux C.coli varie d'un pays à l'autre selon les proportions de 80/20 à 50/50.

Ainsi la présente invention a pour objet une sonde spécifique de l'espèce Campylobacter coli caractérisée en ce qu'elle comporte une séquence d'acides nucléiques ADN ou ARN tirée de la séquence B267 suivante :

```
        10         20         30         40         50
GAXCAXCACC AAAAXCAGCX XXXAAAAXAX XAXXXAXACC AAXXXCCXXA

        60         70         80         90        100
GCAXXGCCAA XXXGCACAGG AGCXCCGXGA ACCAAAGGAA AAXXGGCAGX

       110        120        130        140        150
GAXXXCXXXA GCXXXXXXGG CAXXAXCAGG AGXAAAGGGA CGCAXGGAAA

       160        170        180        190        200
CAACCAXXXC ACCGCXAAAA ACXCCXACAC XXXXGCAAGC AAXAXXGGXX

       210        220        230        240        250
XXAXACAXXG GCACAXXGCA XXXAXXGCXA AXAXGXCXXA XXXCXAAGCC

       260
XXXXXCAAGX AAAGCXX
```

avec X = T pour une séquence ADN

et   X = U pour une séquence ARN

ou la séquence complémentaire inverse en interchangeant X et A d'une part et, C et G d'autre part.

Dans un mode préférentiel de réalisation des sondes, elles sont constituées d'un oligomère marqué comprenant de 15 à 35 de préférence 18 à 24 nucléotides tiré de la séquence B267 mentionnée ci-dessus ou un oligomère complémentaire. On peut citer notamment les séquences

2

```
     CCX XXG GXX CAC GGA GCX CCX G
et   CCA XGC GXC CCX XXA CXC CXG AX.
```

De même l'invention a pour objet comme sonde spécifique de l'espèce <u>Campylobacter coli</u> une séquence d'acides nucléiques comportant une séquence ADN ou ARN tirée de la séquence B-81 suivante :

```
          10         20         30         40         50
AAGCXXACGA XAXAAGCGAG XAXXAXAACG AXGAXXXGAX AGGXXXXXXA

          60         70         80
CXGGGXXGXA GCXXXXCXXX XGAAGAAGCX X
```

avec X = T pour une séquence d'ADN

X = U pour une séquence d'ARN

ou la séquence complémentaire en interchangeant X et A d'une part et, C et G d'autre part.

Avantageusement ces sondes sont constituées par un oligomère marqué de 15 à 35, de préférence 18 à 24 nucléotides tiré de la séquence B81 ci-dessus mentionnée, ou un oligomère complémentaire.

Et, la présente invention a pour objet une sonde spécifique de l'espèce <u>Campylobacter jejuni</u> caractérisée en ce qu'elle comporte une séquence d'acides nucléiques ADN ou ARN tirée de la séquence 15-11 suivante :

```
          10         20         30         40         50
AAGCXXXXXA AAAXGAGGXA GGGXXGAXXX XXCGXXAXXX XCGCXGAXXX

          60         70         80         90        100
XGAXXXXAGG XXXXAXGCCX XGAXCXXCXA AGGCXGCGAG XXXAXAAACG

         110        120        130        140        150
CAACXAAAAA XAGGAXCGCX XGAAGCAGXG AXCAAXCXXX CGCCXACACC

         160        170        180        190        200
AAAGAXAXCA AXAGGXGCAX XXXGXXCXXX GAGXXXXXXA AXACXCCAXX

         210        220        230        240        250
CAXCAAGGGC GXXAGAAACX AXGAXXXXAC AXXXXXGAAG XCCXGCXXGA

         260        270        280        290        300
XCAAGXXCXX GXCXGAXXXX CAAACXCAAC XXAAGXAAAX XXCCAGAAXC

         310        320        330        340        350
XAXXCXGACA CCGCCXXCXX GGAXACCAAA XXCXXXAAAA ACXXXAAXCG

         360        370        380        390        400
CAXXXXXAAG XCCGCXAXXX AGACAAXCAX AAGXAXCGAX CAAAAAAACA

         410        420        430
GGAXXXXXXG GAXAAAXXXX CACAXAAGCX X
```

avec X = T pour une séquence d'ADN

et X = U pour une séquence d'ARN

ou la séquence complémentaire en interchangeant X et A d'une part et, C et G d'autre part.

Dans un mode préférentiel de réalisation la sonde est constituée d'un oligomère marqué de 15 à 35, de préférence 18 à 24 nucléotides, tiré de la séquence 15-11 citée ci-dessus, ou un oligomère complémentaire.

On peut citer en particulier la séquence

GCC XXG AXC XXC XAA GGC XGC GAG

On a donc ainsi sélectionné deux séries de sondes à Campylobacter coli (tirées de B267 et B81) et une série de sondes à Campylobacter jejuni (tirées 15-11). Ces sondes permettent, par hybridation sur colonies, par hybridation sur taches et par hybridation "Southern" de distinguer un Campylobacter coli d'un Campylobacter jejuni.

Par hybridation Southern blot, la spécificité et la sensibilité de ces sondes vis-à-vis de l'ADN de 150 souches différentes de Campylobacter ont été établies.

Les sondes d'acides nucléiques sont bien connues dans l'état de la technique et peuvent être obtenues par diverses voies, notamment par génie génétique ou par synthèse directe manuelle ou automatique.

Ces séquences d'acides nucléiques ont la propriété de s'apparier et de former des hybrides avec les séquences complémentaires d'ADN, le cas échéant préalablement dénaturé, si ceux-ci étaient initialement bicaténaires ou d'ARN. Cette dénaturation peut se faire par incubation en milieu basique ou par élévation de la température du milieu ou par combinaison de ces deux procédés, ou encore par action de microondes.

La détection des hybrides peut se faire par différentes méthodes. La sonde peut être marquée par l'un des procédés connus de l'homme de l'art pour le marquage des sondes d'acides nucléiques. Il peut s'agir d'un marquage radio-actif, par example avec le phosphore 32, ou bien dans le cas d'une sonde froide d'un marquage non radioactif, par exemple enzymatique, comme cela est également connu. Dans certains cas, la sonde n'est pas marquée enzymatiquement lors de son utilisation proprement dite, mais modifiée chimiquement par exemple avec la biotine pour pouvoir être repérée après l'hybridation.

La présente invention a donc également pour objet un procédé de détection et d'identification sélective des infections bactériennes de l'espèce Campylobacter coli ou jejuni, par hybridation avec l'ADN ou l'ARN d'un microorganisme de ladite espèce, caractérisé en ce qu'on hybride deux sondes conformes à la présente invention respectivement spécifiques de C. coli et C. jejuni avec une séquence d'ADN complémentaire appartenant à la souche à identifier, le cas échéant préalablement dénaturée, si elle était initialement bicaténaire ou une séquence d'ARN complémentaire de ladite espèce.

Enfin la présente invention a pour objet une sonde utile dans un procédé d'identification et caractérisation de l'espèce particulière d'un micro-organisme bactérien du genre Campylobacter par méthode "RFLP" consistant en l'analyse par Southern Blot du polymorphisme de restriction du rADN (l'ADN encodant l'ARN ribosomial) présenté par le Campylobacter, caractérisé en ce que sa séquence d'ADN est la suivante :

5'-XGA XCC GAC CGC AGG XXC XCC XA-3'

avec X = T ou U ou sa séquence complémentaire inverse.

Un procédé d'identification et de caractérisation de l'espèce particulière d'un micro-organisme bactérien du genre Campylobacter par une méthode "RFLP" à l'aide de sonde particulièrement approprié est caractérisé en ce que :

1) l'ADN total de la bactérie est digéré par l'enzyme BglII ou les deux enzymes XhoI et BglII,

2) les fragments d'ADN résultant sont séparés par électrophorèse sur gel d'agarose puis

3) dénaturés, transférés et fixés sur une membrane de nitrocellulose ou de nylon,

4) la sonde étant mise à hybrider avec lesdits fragments sur ladite membrane,

5) la relation entre les fragments hybridés à la sonde détectés et leur taille étant la suivante :

- Campylobacter jejuni présente dans un gel d'agarose à 0,4 % trois bandes :

. une vers 4,6 kb, et

. deux bandes de tailles supérieures.

- Campylobacter coli présente toujours une bande aux environs de 0,8 kb et occasionnellement une bande à 2,4 kb ou une bande à 3,6 kb

- Campylobacter laridis présente une bande aux environs de 2,4 kb

- Campylobacter fetus présente une double bande vers 6 kb

- Campylobacter upsaliensis présente deux bandes vers 0,830 kb et 24 kb

Les sondes selon la présente invention sont des sondes oligonucléotidiques caractéristiques des espèces C. jejuni et C. coli. L'intérêt majeur de ces sondes par rapport aux sondes à Campylobacter décrites dans la littérature réside dans leur capacité à pouvoir discriminer entre l'une des deux espèces majeures ( 99 %) de Campylobacters : C. jejuni et C. coli. Par rapport au test biochimique classique, beaucoup plus lent et fiable seulement à 85 % dans les meilleurs cas, ces sondes permettent une identification rapide et précise.

La spécificité et la sensibilité des sondes selon la présente invention ont été testées sur environ 150 souches de Campylobacter et sur environ 100 espèces de la flore commensale. Aucune des sondes à C. jejuni figurant dans la littérature n'a été testée vis-à-vis d'un grand nombre de souches de Campylobacter ou d'espèce de la flore commensale. Les sondes à Campylobacter jejuni mentionnées dans la littérature sont des fragments d'ADN chromosomique alors que les sondes selon l'invention sont des oligonucléotides déterminés.

D'un point de vue médical, il n'a jamais pu être établi clairement une différence de pathogénécité entre C. jejuni et C. coli. Plus précisément les deux études épidémiologiques qui ont été menées à cette fin ont abouti à des résultats contradictoires (The Lancet, January 23, 1988, p 176-77 ; The Lancet, April 23, 1988 p 942-43). Cette lacune provient très probablement du manque de fiabilité et de la difficulté d'interprétation des résultats du test biochimique classique (test de l'hydrolyse de l'hippurate).

La présente invention fournit donc les outils (sondes oligonucléotidiques à C. Jejuni et C. coli) permettant l'identification sélective de C. jejuni et C. coli dans un échantillon biologique, ainsi qu'un procédé de typage de souches de Campylobacter (technique RFLP avec sonde à rDNA) permettant une identification rapide et fiable des différentes espèces de Campylobacter.

Ces outils et procédés devraient permettre aux cliniciens de mettre en évidence une différence de pathogénicité entre les différentes espèces de Campylobacter et par là, leur permettre d'adapter la sanction thérapeutique en fonction de l'espèce de Campylobacter responsable de la maladie.

Le brevet EP-A-0 232 085 décrit des sondes qui hybrident au rARN et qui sont spécifiques du genre Campylobacter mais ne permettent pas d'identifier l'espèce de Campylobacter.

Le brevet international WO 86/04422 décrit des sondes qui sont des fragments d'ADN chromosomiques non caractérisés (non séquencés). Ces sondes ne sont pas spécifiques d'espèces de Campylobacter et ne sont sensibles qu'à 80 %.

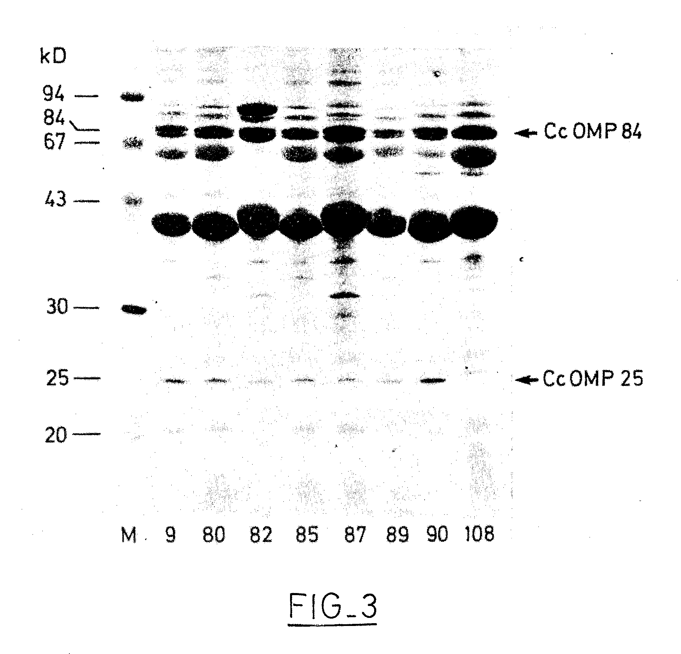

La sonde décrite dans la publication : A specific DNA probe for the identification of Campylobacter jejuni V. Korolik et al. "J. of General Microbiol. 1988 134(2) 521-9 n'est pas spécifique de C. jejuni. En effet, même en conditions sévères d'hybridation, certains C.coli hybrident à cette sonde (figure 3a, pistes 4 et 5). La spécificité de cette sonde vis-à-vis d'un grand nombre d'espèce de la flore commensale n'est pas établie.

Les sondes décrites dans la publication : Molecular cloning of a species-specific DNA probe for C. jejuni P Picken et al. "Mol. and Cellular Probes" 1987 1(3) 245-59 sont des fragments d'ADN chromosomique de 1 Kb minimum. La séquence de ces sondes n'est pas connue. La spécificité des sondes n'est pas établie.

Le procédé RFLP décrit dans "Identification of Campylobacter species by Southern hybridization of genomic DNA" Romaniuk et al. "FEMS Microbiol. letters" 1987 43 (3) 331-5 utilisé par Romaniuk met en oeuvre une sonde à rRNA "spécifique" des Campylobacter. La séquence de la sonde utilisée est différente de celles selon la présente invention.

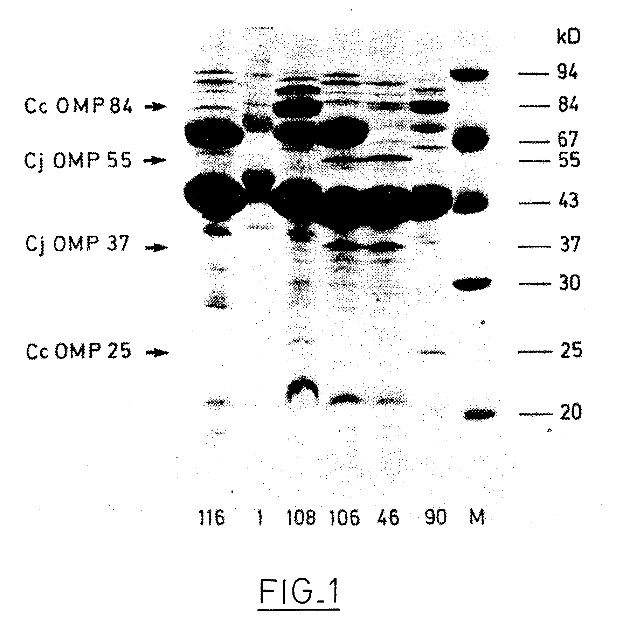

La séquence de la sonde utilisée pour la méthode RFLP selon l'invention est tirée de la séquence de Esherichia Coli. Cette sonde n'est donc pas spécifique du genre Campylobacter mais de part sa nature (séquence conservée entre Esherichia coli et Campylobacter jejuni), elle est très sensible (100 % de sensibilité pour n = 150). Cette sonde permet d'identifier l'espèce particulière d'un Campylobacter (jejuni, coli, laridis, fetus, upsialensis) par analyse du RFLP (XhoI-BglII) du RADN de cette séquence. Le procédé de Romaniuk décrit dans cet article ne permet pas de toujours discriminer entre C. coli et C. jejuni (voir figure 1C, pistes 1 et 6).

Les sondes décrites sont de conceptions totalement différentes, les enzymes de restrictions utilisées sont différentes et les profils de restrictions sont différents.

D'autres avantages et caractéristiques de la présente invention apparaîtrons à la lumière de la description qui va suivre.

Celle-ci est faite en référence aux figures 1 à 6.

Les figures 1 à 3 représentent l'analyse en gel de polyacrylamide du profil des protéines de la membrane externe des Campylobacter. Il s'agit d'une électrophorèse SDS-PAGE (coloration au bleu Coomassie (gradient 14-20 %) des protéines de membrane externe (OMP) provenant de Campylobacter. Les chiffres en bas de chaque colonne identifient les souches. Colone M : masse moléculaire étalon des protéines, en kilodaltons. Cc identifie les protéines OMP caractéristiques de C. coli Cj identifie les protéines OMP caractéristiques de C. jejuni.

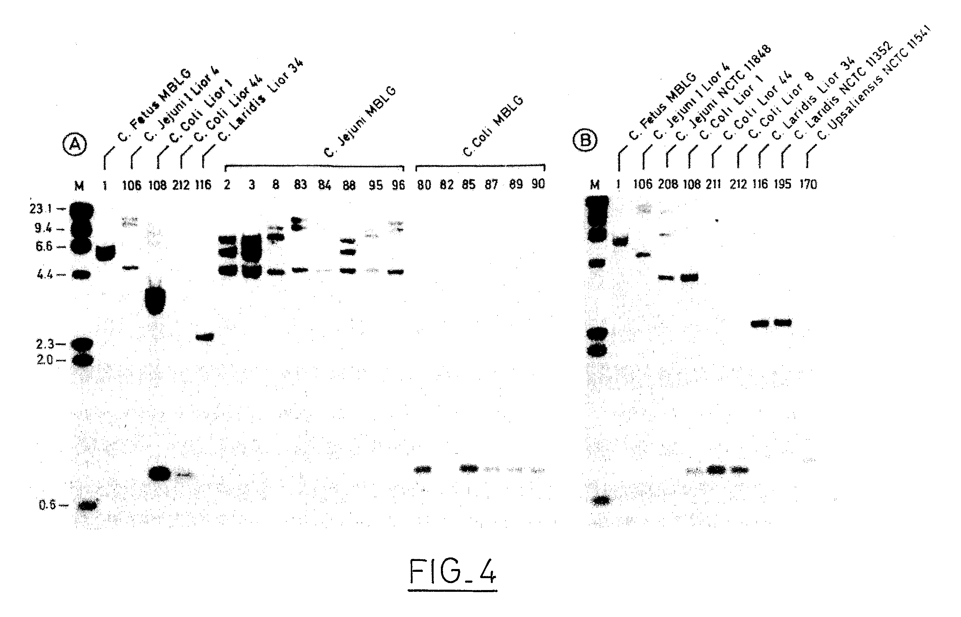

La Figure 4 représente l'analyse par "Southern blot" de l'ADN génomique de Campylobacter coupé par les enzymes XhoI/BglII et hybridé à la sonde complémentaire du 16S rDNA, de séquence

5' TGA TCC GAC CGC AGG TTC TCC TA    3'

Panneau A : l'ADN de Campylobacter coli (souches n° 108, 212, 80, 82, 85, 87, 89, 90), de Campylobacter jejuni (souches n° 106, 83, 84, 96, 8, 95, 2, 3, 88), de Campylobacter fetus (souche n° 1) et de Campylobacter laridis (souche n° 116) à été digéré par la combinaison d'enzymes XhoI - BglII. Les chiffres au-dessus de la photo correspondent aux différentes souches. Les fragments d'ADN ont été transférés sur une membrane de nylon. La membrane a été hybridée à l'aide de la sonde à rADN marquée au [32]P. Le marqueur de poids moléculaire est une digestion HindIII du phage lambda (piste M; les chiffres sur le côté de la photo correspondent à la taille des fragments du marqueur de poids moléculaire).

Panneau B : l'ADN des souches de référence a été digéré par les enzymes XhoI et BglII et analysé tel que décrit au panneau A.

| Souches de référence | | Souches MBLG | |
|---|---|---|---|
| 1 | C. fetus | 2 | C. jejuni |
| 106 | C. jejuni I Lior 4 | 3 | C. jejuni |
| 208 | C. jejuni NCTC 11848 | 8 | C. jejuni |
| 108 | C. coli Lior 1 | 46 | C. jejuni |
| 211 | C. coli Lior 44 | 83 | C. jejuni |
| 212 | C. coli Lior 8 | 84 | C. jejuni |
| 116 | C. laridis Lior 34 | 88 | C. jejuni |
| 195 | C. laridis NCTC 11352 | 95 | C. jejuni |
| 170 | C. upsaliensis NCTC 11541 | 96 | C. jejuni |
| | | 203 | C. jejuni |
| | | 9 | C. coli |
| | | 80 | C. coli |
| | | 82 | C. coli |
| | | 85 | C. coli |
| | | 87 | C. coli |
| | | 89 | C. coli |
| | | 90 | C. coli |

(MBLG : Sigle du laboratoire de Microbiologie à l'Université Catholique de Louvain)

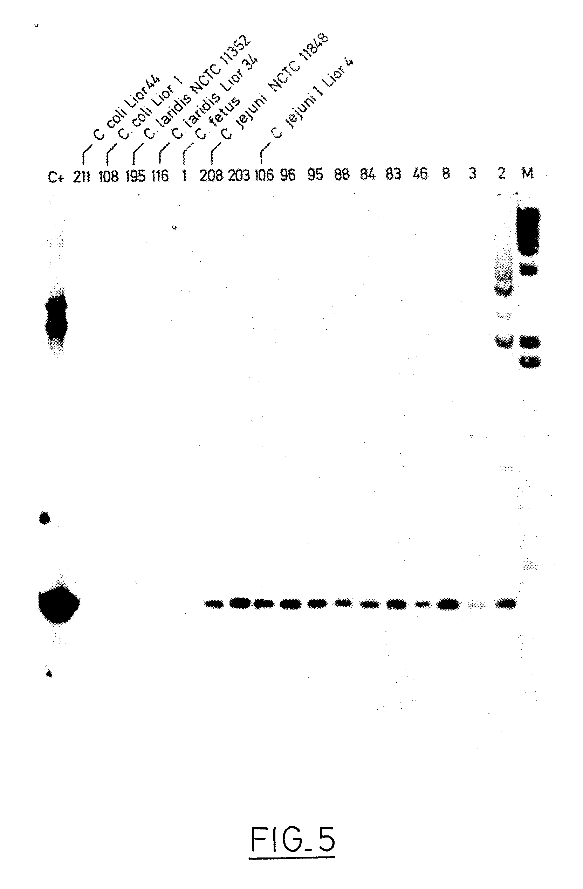

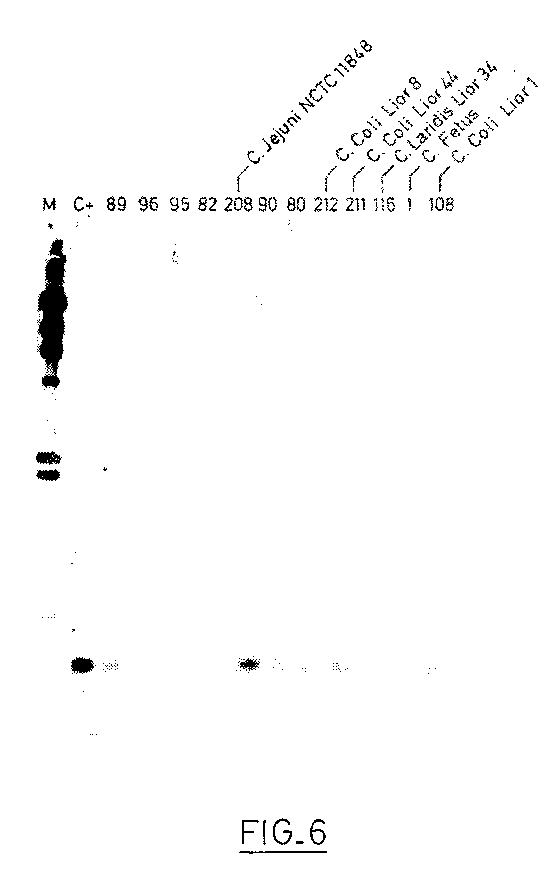

Les figures 5 et 6 représentent des analyses de Southern de l'ADN génomique de Campylobacter, avec

Figure 5 : analyse de l'ADN génomique de Campylobacter digéré par l'enzyme HindIII hybridé avec la sonde 15-11

Figure 6 : analyse d l'ADN génomique de Campylobacter digéré par les enzymes HindIII/Sau3A et hybridé avec la sonde B267.

Exemple 1 : Classification des souches

Les premières étapes de l'invention ont consisté en l'établissement d'une collection de Campylobacter et en la classification des souches de celle-ci. Ces étapes étaient indispensables à la détermination de la spécificité et de la sensibilité des sondes isolées ultérieurement.

En bactériologie classique,la discrimination entre C. coli et C. jejuni est basée uniquement sur le test de l'hydrolyse de l'hippurate. Or, ce test biochimique est généralement reconnu comme n'étant seulement fiable qu'à 85 % dans le meilleur des cas. On a dès lors décidé de suivre deux approches différentes pour la classification des souches : i) la première est basée sur le pourcentage d'homologie entre les ADN totaux des différentes espèces; ii) la deuxième repose sur l'analyse en gel de polyacrylamide du profil des protéines de la membrane externe des Campylobacter (voir figures 1 - 3) (SDS-PAGE des protéines de membrane externe).

Les résultats obtenus par ces deux méthodes (tableau I ci-après) sont en parfaite concordance pour les 25 souches analysées.

Sur les figures 1 à 3 sont concernées les souches suivantes :

Fig. 1 : 116 : C. laridis I Lior 34

1 : C. fetus

108 ; C.coli Lior I

106 : C.jejuni I Lior 4

46 : souche isolée au laboratoire

90 : souche isolée aux Cliniques St Luc, Bruxelles, Belgique.

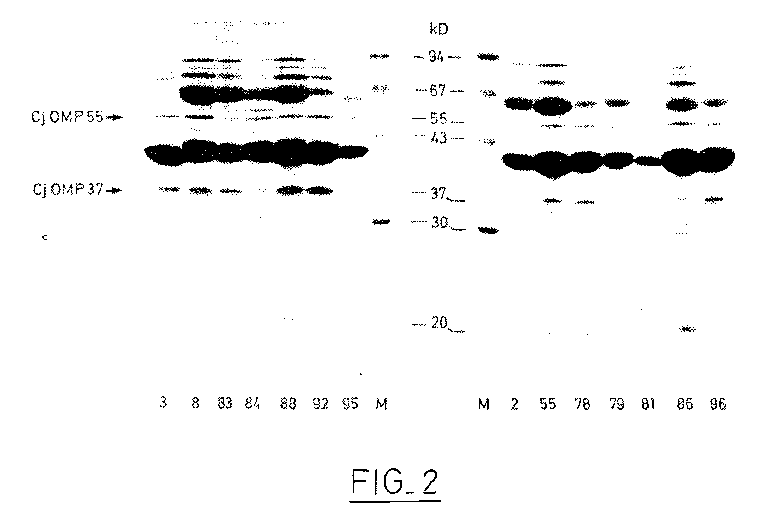

Fig. 2 : 3-8-83-84-88-92-95-2-55-78-79-81-86-96 : souches isolées aux Cliniques St Luc, Bruxelles, Belgique.

Fig. 3 : 9-80-82-85-87-89-90 et 108 : souches isolées aux Cliniques St Luc, Bruxelles, Belgique.

L'analyse des fig. 1 à 3 révèle l'existence de 2 protéines spécifiques de Campylobacter jejuni et de 2 protéines spécifiques de Campylobacter coli, nommées respectivement Cj OMP 55 (PM 55 kDa), Cj OMP 37 (PM : 37 kDa), Cc OMP 84 (PM 84 kDa) et Cc OMP 25 (PM 25 kDa). Ces quatre protéines pourraient dès lors servir à l'élaboration d'un test diagnostic pour le typage des Campylobacters basé, soit sur l'utilisation d'anticorps spécifiques de ces protéines, soit sur l'utilisation de peptides synthétiques correspondant à ces protéines de manière à doser des anticorps circulant dirigés contre ces 4 protéines spécifiques de C. coli et C. jejuni, soit sur des sondes ADN/ARN spécifiques des gènes ou des ARNm encodant ces protéines.

Ces 25 souches ont servi de souche de référence pour la mise au point d'une méthode d'identification des Campylobacter basée sur le polymorphisme (XhoI-BgIII) du rADN (l'ADN qui code pour l'ARN ribosomial). En utilisant comme sonde un oligonucléotide de séquence

5' TGA TCC GAC CGC AGG TTC TCC TA 3'

hybridant à une région très conservée des rADN de <u>Campylobacter</u>, cette méthode permet, par Southern blot, d'identifier les différentes espèces de ce genre. Les 150 souches de notre collection ont été identifiées par cette méthode. Ces mêmes 150 souches ont été utilisées pour la détermination de la spécificité et la sensibilité des sondes à <u>Campylobacter</u> (sondes "B267" et "15-11") selon l'invention. Les résultats de cette étude montrent par ailleurs que l'espèce <u>C.coli</u> est bien plus représentée en Belgique (environ 16 %) que des études antérieures ne l'avaient laissé supposer.

Tableau 1. Discrimination entre <u>Campylobacter jejuni</u> et <u>Campylobacter coli</u> par le test d'hydrolyse de l'hippurate, les profils d'OMP et le pourcentage d'homologie des ADN totaux.

| Souches de référence | Hydrolyse de l'hippurate | Profils OMP | Pourcentage d'homologie avec la souche | |
|---|---|---|---|---|
| | | | <u>C.jejuni</u> I Lior 4 | <u>C.coli</u> Lior 1 |
| 1 <u>C.fetus</u> | n.a. | F | 2 % | 5 % |
| 106 <u>C.jejuni</u> I Lior 4 | + | J | 100 % (R) | 34 % |
| 208 <u>C.jejuni</u> NCTC 11848 | n.i. | n.t. | 65 % | 27 % |
| 108 <u>C.coli</u> Lior 1 | − | C | 31 % | 100 % (R) |
| 211 <u>C.coli</u> Lior 44 | − | C | 29 % | 80 % |
| 212 <u>C.coli</u> Lior 8 | − | C | 38 % | 97 % |
| 116 <u>C.laridis</u> Lior 34 | n.a. | n.a. | 14 % | 10 % |
| 195 <u>C.laridis</u> NCTC 11352 | n.a. | n.a. | 22 % | 11 % |
| Souches testées | | | | |
| 2 | + | J | 115 % | 21 % |
| 3 | − | J | 100 % | 36 % |
| 8 | + | J | 79 % | 28 % |
| 9 | n.i. | C | 47 % | 92 % |
| 46 | + | J | 104 % | 28 % |
| 80 | n.i. | C | 32 % | 69 % |

EP 0 350 392 A2

Tableau 1 (suite)

| Souches testées | Hydrolyse de l'hippurate | Profils OMP | Pourcentage d'homologie avec la souche | |
|---|---|---|---|---|
| | | | C.jejuni I Lior 4 | C.coli Lior 1 |
| 82 | n.i. | C | 43 % | 95 % |
| 83 | n.i. | J | 107 % | 35 % |
| 84 | + | J | 77 % | 31 % |
| 85 | n.i. | C | 38 % | 83 % |
| 87 | n.i. | C | 41 % | 91 % |
| 88 | + | J | 77 % | 34 % |
| 89 | – | C | 38 % | 79 % |
| 90 | – | C | 43 % | 99 % |
| 95 | + | J | 125 % | 46 % |
| 96 | n.i. | J | 94 % | 39 % |
| 203 | n.i. | J | 109 % | 33 % |

J = Campylobacter jejuni    C = Campylobacter coli    F = Campylobacter fetus

n.i. = non identifiable    n.t. = non testé    n.a. = pas applicable

OMP = protéines de membrane externe

R = souche de référence utilisée pour la détermination du pourcentage d'homologie.

Exemple 1A

PREPARATION ET ANALYSE DES PROTEINES DE MEMBRANE EXTERNE (OMP) DE CAMPYLOBACTER

Après 48 heures de culture à 37°C en atmosphère microaérophile (Gaspak anaerocult C Merck), les bactéries sont resuspendues dans un tampon phosphate 0,001 M pH 7. Leur concentration est ajustée à 10 CFU/ml. (CFU = colony forming units).

Ces suspensions sont désintégrées 2 minutes aux ultra-sons (Sonicateur Branson B12) puis centrifugées 20 minutes à 5000 g par aliquot de 3 ml. Les surnageants sont de nouveau centrifugés 90 minutes à 33000 g. Les culots sont resuspendus dans 0,5 ml d'eau distillée additionnée après resuspension de 2 volumes de tampon phosphate 0,001M pH 7 à 2 % en sodium lauryl sarcosinate. Après 30 minutes d'agitation (150 t/min) à température ambiante, ce mélange est centrifugé 90 minutes à 33000 g. Les OMP (culots) récoltés dans 200 μl de tampon d'échantillon (Tris.HCl 50 mM pH 6,8, β-mercaptoéthanol 5 %ov/v, sodium dodecyl sulfate 1 %, glycérol 15 % v/v, Bleu de bromophénol,1,01%) sont soit congelés à -20°C pour conservation soit plongés 5 minutes dans un bain d'eau bouillante en vue d'une analyse par électrophorèse sur un gel vertical de polyacrylamide en gradient (14-20 %) en milieu dénaturant (= SDS - PAGE, acrylamide-bisacrylamide 29:1, SDS 0,4%).

Le volume analysé est de 30 μl, la migration s'effectue sous ampérage constant de 15 mA durant 16 heures. Les protéines sont révélées par coloration au bleu de Coomassie.

Exemple 1B

DESCRIPTION DE LA TECHNIQUE POUR LE TYPAGE DES CAMPYLOBACTERS BASE SUR LE POLYMORPHISME DE RESTRICTION (RFLP) DU rADN

L'ADN total (1 à 2 microgrammes) a été incubé avec 20 unités de chacun des enzymes de restriction XhoI et BglII, pendant 8 à 16 heures à 37°C, dans 40 μl de tampon de restriction (Tris-HCl 37,5 mM, MgCl$_2$ 9,5 mM, DTT 1,25 mM, NaCl 125 mM, BSA 225 μg/ml; pH 7,5). La réaction a été arrêtée par addition de 5 μl de 10xTBE (1xTBE = Tris-HCl 0,05 M, acide borique 0,05 M, EDTA 0,001 M; pH 8,3) contenant 30 % de Ficoll (Pharmacia) et 0,25 % de bleu de bromophénol. Les fragments d'ADN ont été séparés sur un gel d'agarose à 0,8 % (Seakem; FMC) dans du 1xTBE contenant 1 μg/ml de bromure d'ethidium. Après électrophorèse à 25 mA pendant 16 heures dans du 1xTBE contenant 0,1 μg/ml de bromure d'ethidium, le gel a été trempé 15 minutes dans un bain de HCl 0,25 N afin de dépuriner l'ADN. Le gel a ensuite été trempé successivement 30 minutes dans un bain de NaOH 0,5 M - NaCl 1,5 M et 30 minutes dans un bain de Tris-HCl 0,5 M (pH 7,5) - NaCl 1,5 M. Les fragments d'ADN ont été transférés du gel vers une membrane de nylon (Hybond-N; Amersham) en utilisant du 10xSSC (1,5 M NaCl, 0,15 M Na$_3$ citrate) comme tampon de transfert. La membrane a été lavée pendant 5 minutes dans du 2xSSC, séchée pendant une minute, et l'ADN fixé sur la membrane par irradiation aux UV (312 nm).

La membrane a ensuite été préhybridée pendant 2 heures à 50°C dans du 6xSSC contenant 1 % de lait en poudre (Gloria de Nestlé), 2 % de SDS (sodium dodécyl sulfate) et 20 μg/ml d'ADN dénaturé provenant de thymus de veau (Serva). La membrane a ensuite été hybridée dans le même tampon (25 μl/cm$^2$) contenant la sonde ORJ 16Sa à raison de 10$^6$ dpm/ml. Le marquage terminal de la sonde a été réalisé par la T4 kinase utilisant le γ-[$^{32}$P]-ATP comme substrat, selon la méthode de Wallace.

Après 16 heures d'incubation à 50°C, la membrane a été lavée 3 fois pendant 15 minutes dans du tampon 2xSSC - SDS 1 % à 50°C.

La membrane a été séchée à l'air et autoradiographiée à -80°C avec un écran intensificateur pendant 16 à 48 heures (Maniatis et al., 1982).

Exemple 2 : Origine des sondes

La source d'ADN est l'ADN génomique de Campylobacter. L'ADN total d'une souche de C.jejuni et de C.coli a été clivé partiellement par l'enzyme de restriction Sau3A de façon à générer des fragments d'ADN d'environ 4 Kb. Les différents fragments de restriction de C. jejuni ont été clonés dans le vecteur pBR322 et les fragments de C.coli dans le vecteur pUC9, générant ainsi une banque génomique de chaque espèce. Les sondes à C.coli et à C.jejuni ont été isolées de ces banques après criblage de celles-ci.

Exemple 3 : Critères de sélection des sondes

Il existe deux critères qui permettent d'évaluer la qualité d'une sonde : la spécificité et la sensibilité. Lorsqu'une sonde est caractéristique d'une espèce, la spécificité consiste en la propriété de cette sonde de s'hybrider uniquement à l'ADN (ou à l'ARN) des souches appartenant à cette espèce. La sensibilité d'une telle sonde réside dans la capacité de cette sonde d'hybrider à l'ADN (ou l'ARN) de toutes les souches de cette espèce.

Exemple 4 : Criblage des banques génomiques

Il a été mis au point selon l'invention un protocole qui nous a permis d'isoler, par hybridation différentielle, des clones spécifiques de chaque espèce. Selon notre protocole, trois copies de chaque clone de la banque génomique ont été hybridées respectivement avec l'ADN total de C.coli, C.jejuni et C.laridis. Pour chaque banque, les clones hybridant à l'ADN total homologue (c'est-à-dire l'ADN dont ils sont issus) mais n'hybridant pas à l'ADN total des deux autres espèces hétérologues sont été retenus. L'ADN plasmidique de cha cun de ces clones a été purifié et retesté comme décrit ci-dessus. Les clones sélectionnés par ces deux étapes de criblage ont été hybridés avec l'ADN total de 25 souches de Campylobacter caractérisées comme décrit à l'exemple 1. Les clones les plus sensibles et les plus spécifiques dans ce test (appelés selon l'invention 15-11, B267 et B81) ont été retenus pour un 4ième criblage par hybridation sur colonies. Les 25 souches de Campylobacter ont été cultivées sur deux séries de membranes de nylon (Hybond-N de Amersham) déposées sur du milieu sélectif pour Campylobacter. Après culture et traitement des membranes selon les recommandations du constructeur, une série de membranes a été hybridée avec la sonde B267 et l'autre avec la sonde 15-11.

Toutes les souches classées comme C.coli ou comme C.jejuni hybrident plus fortement à B267 ou à 15-11 respectivement. Les souches classées comme C.fetus ou C.laridis hybrident plus faiblement et de manière identique avec les deux sondes.

Exemple 5 : Détermination de la spécificité et de la sensibilité des sondes

L'étude de la spécificité et de la sensibilité d'une sonde se fait généralement soit par hybridation sur colonies, soit par hybridation sur taches. Dans le premier cas, les sondes sont hybridées avec un grand nombre de souches sans purifier l'ADN de celles-ci. Lorsque l'ADN des souches est purifié, l'étude de la spécificité et de la sensibilité peut se faire par hybridation sur taches ("dot hybridization") ou par "Southern blot".

L'étude de la spécificité (au sein du genre Campylobacter) et de la sensibilité des sondes B267 et 15-11 repose sur l'analyse, par Southern blot, du polymorphisme de restriction des Campylobacter. Cette technique présente, outre la capacité de vérifier la sensi bilité d'une sonde, la particularité de souligner la spécificité de celle-ci, en indiquant, pour chaque souche analysée, la taille du(des) fragment(s) d'ADN génomique qu hybride(nt) à la sonde.

L'étude de la spécificité des sondes B267 et 15-11 a été réalisée par hybridation Southern de l'ADN génomique des 150 souches de Campylobacter classées précédemment.

On a ainsi sélectionné une sonde à C. jejuni (un fragment HindIII de 431 bp provenant du clone 15-11) qui hybride avec l'ADN de toutes les souches C. jejuni au niveau d'un fragment HindIII de taille égale à celle de la sonde (fig. 5). Cette sonde ne donne pas d'hybridation avec l'ADN des souches des autres espèces. Il ne semble donc pas exister de polymorphisme de restriction (restriction fragment lenght polymorphism) pour cette sonde au sein de l'espèce C. jejuni. Ceci indique que notre sonde reconnaît une région spécifique et conservée de l'espèce C. jejuni.

Pour l'expérience reproduite sur la figure 5, 2 µg d'ADN de Campylobacter coli (souches 211 et 108), Campylobacter laridis (souches 195 et 116), Campylobacter fetus (souche 1), et Campylobacter jejuni (souches 208 à 2 : colonnes 7 à 18)ont été digérées avec HindIII. La sonde spécifique de C. jejuni était biotinylée. L'ADN de lambda digérée par HindIII est utilisé comme marqueur de poids moléculaire (colonne M ). Un vecteur-sonde mon marqué, digéré par HindIII, a été utilisé comme contrôle positif (colonne C).

On a également isolé deux sondes à C.coli qui possèdent les mêmes caractéristiques de spécificité que la sonde C.jejuni. Il s'agit d'un fragment HindIII de 81 bp (sonde B81) et d'un fragment HindIII-Sau3A de 267 bp (sonde B267). Ce dernier hybride avec l'ADN de toutes les souches C.coli au niveau d'un fragment HindIII-Sau3A de 267 bp. Cette sonde ne donne pas d'hybridation avec l'ADN des souches des autres espèces (fig.6).

Pour l'expérience reproduite sur la figure 6, 2 µg d'ADN de Campylobacter coli (souches n° 89,82,90,80,212, 211, 108), Campylobacter fetus (souche 1), C.laridis (souche 116), et C.jejuni (souches 96, 95, 208) ont été digérés avec HindIII-Sau3A. La sonde spécifique de C.coli (B267) était biotinylée. L'ADN de lambda digéré par HindIII a été utilisé comme marqueur de poids moléculaire (colonne M). Une sonde électro-éluée non marquée (100 pg) a été utilisée comme contrôle positif (colonne C+).

La spécificité et la sensibilité de la sonde à C.coli B81 ont également été établies.

EXEMPLE 6 : Sous-clonages et séquençage

Les sondes retenues (B81,B267 et 15-11) ont été sous-clonées dans le vecteur phagique monocaténaire M13 et les séquences nucléotidiques ont été déterminées.

EXEMPLE 7 : Détermination de la spécificité et de la sensibilisation des sondes

Deux oligonucléotides, correspondant à la sonde B267, appelés OCC267a et OCC267b, et un oligonucléotide correspondant à la sonde 15-11 (appelé OCJ1511a) ont été synthétisés et testés en hybridation sur tâches et hybridation sur colonies. Les résultants du calcul de spécificité et sensibilité, basé sur l'hybridation sur tâches et sur colonies de ces sondes avec 115 souches de Campylobacter (96 C. jejuni et 19 C. coli) sont repris dans le tableau suivant :

|          | Spécificité | Sensibilité |
|----------|-------------|-------------|
| OCC267a  | 97 %        | 100 %       |
| OCC267b  | 97 %        | 95 %        |
| OCJ1511a | 95 %        | 100 %       |

OCC267a

Séquence : 5' > CCT TTG GTT CAC GGA GCT CCT G < 3'

22 BASES

OCC267b

Séquence : 5' > CCA TGC GTC CCT TTA CTC CTG AT < 3'

23 BASES

OCJ1511a

Séquence : 5' > GCC TTG ATC TTC TAA GGC TGC GAG < 3'

24 BASES

Ces oligonucléotides n'hybrident pas avec les souches de la flore commensale énumérées ci-après. Les souches suivantes ont été testées par hybridation sur colonies :

1. Enterobactéries
Enterobacter aerogenes
Enterobacter agglomerans
Enterobacter cloacae
Hafnia alvei
Alcalescens dispar
Salmonella typhi
Salmonella para typhi A
Solmonella para typhi B
Salmonella infantis C
Salmonella enteritidis D
Proteus vulgaris
Proteus mirabilis
Proteus morganii
Citrobacter freundii
Citrobacter amalonaticus (2)
Citrobacter diversus
Providencia rettgeri
Providencia alcalifaciens
Providencia stuartii
Serratia liquefaciens
Serratia marcescens
Escherichia coli
Edwardsiella
Klebsiella oxytoca
Klebsiella pneumoniae
Klebsiella aerogenes
Shigella flexneri
Shigella sonnei
Shigella dysenteriae
Shigella flexneri 1
Shigella flexneri 1a
Shigella flexneri 2a
Shigella flexneri 4a

Shigella flexneri 6
Shigella boydii 1
Shigella boydii 2
Shigella boydii 14
Shigella dysenteriae 2
Shigella dysenteriae 3
Yersinia enterocolitica W22703
Yersinia enterocolitica E439:80 CaD 0:9
Yersinia enterocolitica E439:80 CaI 0:9
Yersinia enterocolitica 708-82 CaD 0:5, 27
Yersinia enterocolitica W22708 CaD 0:9
Yersinia enterocolitica W22708 CaI 0:9
Yersinia enterocolitica W836 CaD 0:5, 27
Yersinia enterocolitica E104/81 CaI 0:10
Yersinia enterocolitica W845 CaD 0:3
Yersinia enterocolitica IP38 CaD 0:2
Yersinia enterocolitica T1758 CaD 0:5
Yersinia enterocolitica J35 CaI 0:5
Yersinia enterocolitica E334/80 CaI 0:6, 30
Yersinia enterocolitica W852 CaI 0:47
Yersinia enterocolitica IP106 CaI 0:7,8
Yersinia enterocolitica E301-82 CaI 0:41


2. Anaerobies
Clostridium difficile
Clostridium subterminale
Clostridium sporogenes
Clostridium bifermentans
Fusobacterium nucleatum
Fusobacterium mortiferum
Bacteroïdes fragilis


3. Coques gram +
Enterococcus faecalis
Enterococcus faecium
Streptococcus bovis
Streptococcus milleri
Staphylococcus aureus


4. Fermentants oxydase +
Aeromonas hydrophyla
Plesiomonas
Vibrio cholerae
Vibrio alginolyticus


5. Non fermentants
Pseudomonas aeruginosa
Pseudomonas fluorescens
Pseudomonas maltophylia
Alcaligenes faecalis
Alcaligenes denitrificans
Acinetobacter


6. Escherichia coli avec plasmides

28TAM1 (Col VBM260)
CGSC 6059 (F)
711 (p307)
3272 (pGC1)
J53 (pGC200)
J53 (RP4)
J53 (Rsa)
C600 (RSF1010)

7. Contrôles Campylobacter
Campylobacter fetus (souche 1)
Campylobacter laridis (souche 116)
Campylobacter laridis (souche 195)
Campylobacter upsaliensis (souche 170)

Mode opératoire :

Cent et quinze souches ($10^7$ cellules/souches) de Campylobacter classées antérieurement ont été cultivées sur trois séries de membranes de nylon (Hybond-N de Amersham) déposées sur du milieu sélectif pour Campylobacter. Après 16 h de culture à 42°C en atmosphère microaérophile, les membranes ont été séchées à l'air, déposées successivement 15 minutes sur un coussin de NaOH 1N-EtOH 20 %, 3 x 1 minutes sur un coussin de Tris-HCl 1M (pH 7,5). Les membranes ont été séchées à l'air pendant 30 minutes et l'ADN des bactéries fixé sur la membrane par irradiation aux UV (312 nm). Les membranes ont ensuite été préhybridées pendant 1 heure à 60 °C dans du 2xSSC contenant 1% de lait en poudre (Gloria de Nestlé), 0,1 % de SDS et 20 µg/ml d'ADN dénaturé provenant de thymus de veau (Serva). Chaque série de membrane a ensuite été hybridée dans un tampon de même composition contenant une des trois sondes OCC267a, OCC267b et OCJ1511a à raison de $10^6$ dpm/ml. Le marquage terminal de chaque sonde a été réalisé en utilisant la T4 kinase et le $\gamma$-$^{32}$P-ATP selon la méthode de Wallace.

Après 16 heures d'incubation à 60°C, chaque membrane a été lavée 3 fois pendant 10 minutes dans du tampon 2xSSC - SDS 0,1 % à 60°C. Les membranes ont été séchées à l'air et auto-radiographiées à -80°C pendant 16 heures.

Les résultants de l'hybridation de chacune des sondes ont été comparés aux résultats du typage de ces 115 souches par analyse du polymorphisme de restriction XhoI-BgII des rADN de ces souches.

Le tableau ci-après illustre, pour 115 souches testées, le calcul de la spécificité et de la sensibilité de la sonde OCC267b

## Campylobacter coli identifiés par
### analyse du RFLP

| | | + | − | |
|---|---|---|---|---|
| hybridation à la sonde OCC267b | + | 18 | 3 | 21 |
| | − | 1 | 93 | 94 |
| | | 19 | 96 | 115 |

sensibilité : $\dfrac{18}{19}$ x 100 = 95%

spécificité : $\dfrac{93}{96}$ x 100 = 97%

**Revendications**

1 - Sondes d'acides nucléiques ADN ou ARN consistant en une séquence déterminée d'acides nucléiques d'ADN ou ARN respectivement, ladite séquence étant marquée, utile pour détecter de manière spécifique l'une des espèces bactériennes du genre Campylobacter.

2 - Sonde selon la revendication 1, caractérisée en ce qu'elle est spécifique de l'espèce Campylobacter.coli.

3 - Sonde selon la revendication 1, caractérisée en ce qu'elle est spécifique de l'espèce Campylobacter jejuni.

4 - Sonde selon la revendication 2, caractérisée en ce qu'elle comporte une séquence d'acides nucléiques ADN ou ARN tirée de la séquence B267 suivante :

```
        10          20          30          40          50
GAXCAXCACC  AAAAXCAGCX  XXXAAAAXAX  XAXXXAXACC  AAXXXCCXXA

        60          70          80          90          100
GCAXXGCCAA  XXXGCACAGG  AGCXCCGXGA  ACCAAAGGAA  AAXXGGCAGX

        110         120         130         140         150
GAXXXCXXXA  GCXXXXXXGG  CAXXAXCAGG  AGXAAAGGGA  CGCAXGGAAA

        160         170         180         190         200
CAACCAXXXC  ACCGCXAAAA  ACXCCXACAC  XXXXGCAAGC  AAXAXXGGXX

        210         220         230         240         250
XXAXACAXXG  GCACAXXGCA  XXXAXXGCXA  AXAXGXCXXA  XXXCXAAGCC

        260
XXXXXCAAGX  AAAGCXX
```

avec X = T pour une séquence ADN

et   X = U pour une séquence ARN

ou la séquence complémentaire en interchangeant X et A d'une part et, C et G d'autre part.

5 - Sonde selon la revendication 2, caractérisée en ce qu'elle comporte une séquence d'acides nucléiques ADN ou ARN tirée de la séquence B81 suivante :

```
        10          20          30          40          50
AAGCXXACGA  XAXAAGCGAG  XAXXAXAACG  AXGAXXXGAX  AGGXXXXXXA

        60          70          80
CXGGGXXGXA  GCXXXXCXXX  XGAAGAAGCX  X
```

avec X = T pour une séquence d'ADN

    X = U pour une séquence d'ARN

ou la séquence complémentaire en interchangeant X et A d'une part et, C et G d'autre part.

6 - Sonde selon la revendication 3, caractérisée en ce qu'elle comporte une séquence d'acides nucléiques ADN ou ARN tirée de séquence 15-11 suivante :

15

```
          10         20         30         40         50
AAGCXXXXXA AAAXGAGGXA GGGXXGAXXX XXCGXXAXXX XCGCXGAXXX

          60         70      ·  80         90        100
XGAXXXXAGG XXXXAXGCCX XGAXCXXCXA AGGCXGCGAG XXXAXAAACG

         110        120        130        140        150
CAACXAAAAA XAGGAXCGCX XGAAGCAGXG AXCAAXCXXX CGCCXACACC

         160        170        180        190        200
AAAGAXAXCA AXAGGXGCAX XXXGXXCXXX GAGXXXXXXA AXACXCCAXX

         210        220        230        240        250
CAXCAAGGGC GXXAGAAACX AXGAXXXXAC AXXXXXGAAG XCCXGCXXGA

         260        270        280        290        300
XCAAGXXCXX GXCXGAXXXX CAAACXCAAC XXAAGXAAAX XXCCAGAAXC

         310        320        330        340        350
XAXXCXGACA CCGCCXXCXX GGAXACCAAA XXCXXXAAAA ACXXXAAXCG

         360        370        380        390        400
CAXXXXXAAG XCCGCXAXXX AGACAAXCAX AAGXAXCGAX CAAAAAAACA

         410        420        430
GGAXXXXXXG GAXAAAXXXX CACAXAAGCX X
```

avec X = T pour une séquence d'ADN

et    X = U pour une séquence d'ARN

ou la séquence complémentaire en interchangeant X et A d'une part et C et G d'autre part.

7 - Sonde selon l'une des revendications 4 à 6, caractérisée en ce qu'elle est constituée d'un oligomère marqué de 15 à 35, de préférence 18 à 24 nucléotides, tiré de la séquence citée, ou un oligomère complémentaire.

8 - Sonde selon les revendications 4 et 7, caractérisée en ce qu'elle comporte la séquence

CCX XXG GXX CAC GGA GCX CCX G

avec X = T pour une séquence d'ADN

      X = U pour une séquence d'ARN

ou la séquence complémentaire en interchangeant X et A d'une part et C et G d'autre part.

9 - Sonde selon les revendications 4 et 7, caractérisée en ce qu'elle comporte la séquence

CCA XGC GXC CCX XXA CXC CXG AX

avec X = T pour une séquence d'ADN

      X = U pour une séquence d'ARN

ou la séquence complémentaire en interchangeant X et A d'une part et C et G d'autre part.

10 - Sonde selon les revendications 6 et 7, caractérisée en ce qu'elle comporte la séquence

GCC XXG AXC XXC XAA GGC XGC GAG

avec X = T pour une séquence d'ADN

      X = U pour une séquence d'ARN

ou la séquence complémentaire en interchangeant X et A d'une part et C et G d'autre part.

11 - Procédé de détection et d'identification sélective des infections bactériennes de l'espèce Campylobacter coli ou jejuni, caractérisé en ce qu'on hybride deux sondes respectivement spécifiques de C. coli et C. jejuni selon l'une des revendications précédentes avec une séquence d'ADN complémentaire appartenant à la souche à identifier, le cas échéant, dénaturée si elle était initialement bicaténaire, ou une séquence d'ARN complémentaire.

12 - Sonde selon la revendication 1 utile dans un procédé d'identification et détection de l'espèce particulière d'un micro-organisme bactérien du genre Campylobacter par méthode "RFLP" consistant en l'analyse par Southern Blot du polymorphisme de restriction du rADN présenté par le Campylobacter, caractérisée en ce que sa séquence est la suivante :

```
5'-XGA XCC GAC CGC AGG XXC XCC XA-3'
avec X = T ou U ou la séquence complémentaire.
```

13 - Procédé d'identification et de détection de l'espèce particulière d'un micro-organisme bactérien du genre Campylobacter par une méthode "RFLP" à l'aide de la sonde selon la revendication 12, caractérisé en ce que

1) l'ADN total de la bactérie est digéré par l'enzyme BglII ou les deux enzymes XhoI et BglII,
2) les fragments d'ADN résultants sont séparés par électrophorèse sur gel d'agarose puis
3) dénaturés, transférés et fixés sur une membrane de nitrocellulose ou de nylon,
4) la sonde étant mise à hybrider avec lesdits fragments de ladite membrane,
5) la relation entre les fragments hybridés à la sonde détectés et leur taille étant la suivante :
- Campylobacter jejuni présente dans un gel d'agarose à 0,4 % d'agarose trois bandes :
. une vers 4,6 kb, et
. deux bandes de tailles supérieures.
- Campylobacter coli présente toujours une bande aux environs de 0,8 kb et occasionnellement une bande à 2,4 kb ou une bande à 3,6 kb
- Campylobacter laridis présente une bande aux environs de 2,4 kb
- Campylobacter fetus présente une double bande vers 6 kb
- Campylobacter upsaliensis présente deux bandes vers 0,830 kb et 24 kb.

Cc OMP 84 →
Cj OMP 55 →
Cj OMP 37 →
Cc OMP 25 →

kD
— 94
— 84
— 67
— 55
— 43
— 37
— 30
— 25
— 20

116   1   108  106   46   90   M

EP 0 350 392 A2

<u>FIG_1</u>

kD

— 94 —

— 67 —

CjOMP55 →                — 55 —

— 43 —

CjOMP37 →                — 37 —

— 30 —

— 20 —

3   8   83  84  88  92  95   M          M   2   55  78  79  81  86  96

FIG_2

FIG_3

FIG_4

EP 0 350 392 A2

FIG_5

FIG_6